# EUROPÄISCHE PATENTANMELDUNG

(11) **EP 2 338 485 A1**
(43) Veröffentlichungstag der Anmeldung: **29.06.2011**
(21) Anmeldenummer: 09179117.8
(22) Anmeldetag: 14.12.2009
(51) Int. Cl.: A61K 31/4035, A61P 29/00

(54) **Substituierte 1,3-Dioxoisoindoline als Arzneimittel**

(71) Anmelder: Grünenthal GmbH, 52099 Aachen (DE)
(72) Erfinder: Die Erfindernennung liegt noch nicht vor
(74) Vertreter: Michalski Hüttermann & Partner Patentanwälte

(57) **Zusammenfassung**

Die Erfindung betrifft ein Arzneimittel enthaltend wenigstens ein substituiertes 1,3-Dioxoisoindolin (I) sowie die Verwendung dieser Verbindungen zur Herstellung von Arzneimitteln.

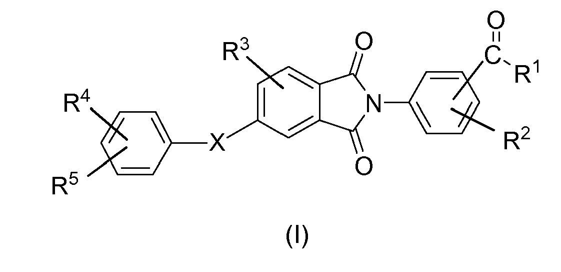

## Beschreibung

Die Erfindung betrifft die Verwendung von substituierten 1,3-Dioxoisoindolinen als Arzneimittel.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Der Einsatz klassischer Opioide in der Schmerztherapie wird durch die bekannten Nebenwirkungen (z.B. Atemdepression, Erbrechen, Sedierung, Obstipation, Toleranzentwicklung) wie auch die geringere Wirksamkeit bei neuropathischen oder inzidentiellen Schmerzen limitiert. Auf zellulärer Ebene erreichen die Opioide wie auch partielle Opioid-Agonisten ihre analgetische Wirkung durch Bindung an membranständige Rezeptoren, die zur Familie der sogenannten G-Proteingekoppelten Rezeptoren gehören.

Aufgrund der dargestellten Unzulänglichkeiten sind Bemühungen zur Identifizierung weiterer Zielproteine für analgetisch wirksame Substanzen neben G-Proteingekoppelten Rezeptoren gestartet worden, wie z.B. differenzielle Genexpressionsanalysen zwischen hyperalgetischen oder allodynischen Mammaliae und gesunden Individuen zur Identifizierung schmerzregulierter Gene (siehe z.B. WO 03/016475).

Im Zuge dieser Bemühungen sind in verschiedenen Schmerzmodellen die vesikulären Glutamat-Transporter (vGLUTs) in dorsalen Wurzelganglien der Ratte als differenziell exprimiert gefunden worden, was z.B. in der WO 2002/0101394 beschrieben wird. Auch in einer weiteren Studie ist ein vesikulärer Glutamat-transporter als differenziell exprimiert im Rückenmark bei zwei chronischen Schmerzstimuli beschrieben (EP 1 284 298).

Die vesikulären Glutamattransporter vGLUT1 und vGLUT2 sind aber nicht nur im Zusammenhang mit der Indikation Schmerz von Bedeutung, sondern spielen auch bei einer Vielzahl weiterer physiologischer und pathophysiologischer Prozesse eine Rolle.

Es besteht daher ein Bedarf an Arzneimitteln enthaltend wenigstens eine Verbindung zur Behandlung und/oder Prophylaxe von medizinischen Indikationen, welche zumindest teilweise durch vGLUT1 und/oder vGLUT2 vermittelt werden, nicht nur im Hinblick auf die Affinität an vGLUT1 und/oder vGLUT2 als solche *(potency, efficacy).*

So kann es vorteilhaft sein, die metabolische Stabilität, die Löslichkeit in wässrigen Medien oder die Permeabilität der Verbindungen zu verbessern. Diese Faktoren können sich günstig auf die orale Bioverfügbarkeit auswirken oder können das PK/PD (Pharmakokinetik/Pharmakodynamik)-Profil verändern, was z.B. zu einer günstigeren Wirkdauer führen kann.

Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind. Zudem sollten die Verbindungen auch keine Problematiken hinsichtlich Toxizität und Carcinogenität aufweisen.

Eine Aufgabe der Erfindung bestand daher darin, neue Verbindungen als Arzneimittel zur Verfügung zu stellen, welche Vorteile gegenüber den Verbindungen des Standes der Technik aufweisen und sich zur Behandlung und/oder Prophylaxe von Störungen oder Krankheiten eignen, welche zumindest teilweise durch vesikuläre Glutamat-Transporter 1 und/oder 2 (vGLUT1 und/oder vGLUT2) vermittelt werden.

Diese Aufgabe wird durch den Gegenstand der unabhängigen Patentansprüche gelöst. Vorteilhafte Ausführungsformen sind in den abhängigen Patentansprüchen wiedergegeben.

Überraschenderweise wurde nun gefunden, dass die substituierten Verbindungen der nachstehend angegebenen allgemeinen Formel (I) eine ausgezeichnete Affinität zu den vesikulären Glutamat-Transportern 1 und/oder 2 (vGLUT1 und/oder vGLUT2) aufweisen und sich daher insbesondere als pharmakologische Wirkstoffe in Arzneimitteln zur Prophylaxe und/oder Behandlung von Störungen oder Krankheiten eignen, die zumindest teilweise durch vGLUT1 und/oder vGLUT2 vermittelt werden. Insbesondere wurde überraschend gefunden, dass sich die substituierten Verbindungen der nachstehend angegebenen allgemeinen Formel (|) in Arzneimitteln zur Regulation der vesikulären Glutamat-Transporter 1 und/oder 2 (vGLUT1 und/oder vGLUT2), vorzugsweise zur Hemmung und/oder zur Stimulation der vesikulären Glutamat-Transporter 1 und/oder 2 (vGLUT1 und/oder vGLUT2) eignen, d.h. sie üben eine agonistische oder antagonistische Wirkung, vorzugsweise jedoch eine antagonistische Wirkung aus.

Ein Gegenstand der vorliegenden Erfindung ist daher wenigstens eine substituierte Verbindung der allgemeinen Formel (I) 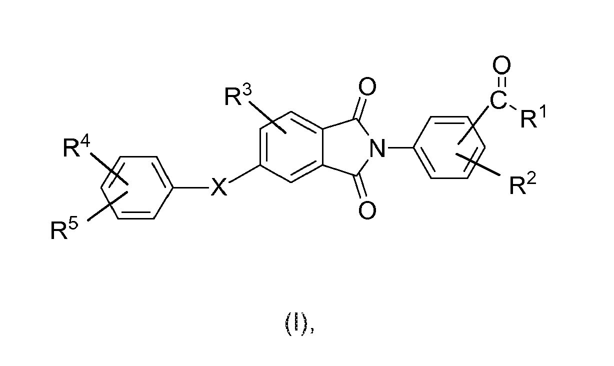 worin
X für -C(=O)NH-, -NH(C=0)- oder für 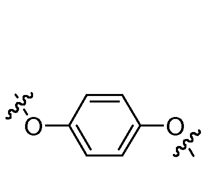 steht;
R¹ für OR⁶. oder NR⁶R⁷ steht,
   wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
R², R³, R⁴ und R⁵ jeweils unabhängig voneinander für H; F; Cl, Br; l; OR⁸; SR⁸; C(=O)OR⁸; CN; CF_{3;} NO₂; NR⁸R⁹; C₁₋₁₀-Alkyl, gesättigt oder ungesättigt, verzweigt oder unverzweigt, unsubstituiert oder einfach oder mehrfach substituiert; C₃-₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen,
   wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
oder, wenn R⁴ und R⁵ an einander benachbarte Kohlenstoffatome gebunden sind, R⁴und R⁵ gemeinsam für die Gruppe -C(=O)-NR¹⁰-C(=O)- stehen können,
   wobei R¹⁰ für C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; steht;
worin "Alkyl substituiert", "Heterocyclyl substituiert" und "Cycloalkyl substituiert" an den entsprechenden Resten für die Substitution eines oder mehrerer Wasserstoffatome jeweils durch Substituenten unabhängig voneinander ausgewählt aus der Gruppe bestehend aus F; Cl; Br; l; NO₂; CN; =O; CF₃; C₁₋₈-Alkyl; Aryl, Heteroaryl; C₃₋₁₀-Cycloalkyl; Heterocyclyl; C(=O)OH;
   C(=O)O-C₁₋₈-Alkyl; C(=O)O-C₃₋₁₀-Cycloalkyl; C(=O)O-Aryl; C(=O)O-Heteroaryl;
   C(=O)NH₂; C(=O)NH-C₁₋₈-Alkyl; C(=O)NH-C₃₋₁₀-Cycloalkyl; C(=O)NH-Aryl;
   C(=O)NH-Heteroaryl;C(=O)N(C₁₋₈-Alkyl)₂;C(=O)N(C₃₋₁₀-Cycloalkyl)₂;
   C(=O)N(Aryl)_{2;}C(=O)N(Heteroaryl)₂;C(=O)N(C₁₋₈-Alkyl)(C₃₋₁₀-Cycloalkyl);
   C(=O)N(C₁₋₈-Alkyl)(Aryl);C(=O)N(C₁₋₈-Alkyl)(Heteroaryl);C(=O)N(C₃₋₁₀-Cycloalkyl)(Aryl), C(=O)N(C₃₋₁₀-Cycloalkyl)(Heteroaryl);
   C(=O)N(Aryl)(Heteroaryl); OH; OCF₃; O-C₁₋₈-Alkyl; O-C₃₋₁₀-Cycloalkyl; O-Aryl;
   O-Heteroaryl; O-C(=O)-C₁₋₈-Alkyl; O-C(=O)-C₃₋₁₀-Cycloalkyl; O-C(=O)-Aryl; O-C(=O)-Heteroaryl; SH; SCF₃; S-C₁₋₈-Alkyl; S- C₃₋₁₀-Cycloalkyl; S-Aryl; S-Heteroaryl; NH₂; NH-C₁₋₈-Alkyl; NH-C₃-₁₀-Cycloalkyl; NH-Aryl; NH-Heteroaryl;
   N(C₁₋₈-Alkyl)₂; N(C₃₋₁₀-Cycloalkyl)₂; N(Aryl)₂; N(Heteroaryl)₂; N(C₁₋₈-Alkyl)(C₃₋₁₀-Cycloalkyl); N(C₁₋₈-Alkyl)(Aryl); N(C₁₋₈-Alkyl)(Heteroaryl);
   N(C₃₋₁₀-Cycloalkyl)(Aryl); N(C₃₋₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl; steht;
worin "Aryl substituiert" und "Heteroaryl substituiert" an den entsprechenden Resten für die Substitution eines oder mehrerer Wasserstoffatome jeweils durch Substituenten unabhängig voneinander ausgewählt aus der Gruppe bestehend aus F; Cl; Br; l; NO₂; CN; CF₃; C₁₋₈-Alkyl; Aryl; Heteroaryl; C₃₋₁₀-Cycloalkyl; Heterocyclyl; C(=O)OH; C(=O)O-C₁₋₈-Alkyl; C(=O)O-C₃-₁₀-Cycloalkyl; C(=O)O-Aryl; C(=O)O-Heteroaryl; C(=O)NH₂; C(=O)NH-C₁₋₈-Alkyl;
   C(=O)NH-C₃₋₁₀-Cycloalkyl; C(=O)NH-Aryl; C(=O)NH-Heteroaryl;
   C(=O)N(C₁₋₈-Alkyl)₂; C(=O)N(C₃₋₁₀-Cycloalkyl)_{;} C(=O)N(Aryl)₂;
   C(=O)N(Heteroaryl)₂; C(=O)N(C₁₋₈-Alkyl)(C₃₋₁₀-Cycloalkyl)_{;}
   C(=O)N(C₁₋₈-Alkyl)(Aryl); C(=0)N(C₁₋₈-Alkyl)(Heteroaryl); C(=O)N( C₃₋₁₀-Cycloalkyl)(Aryl); C(=O)N(C₃₋₁₀-Cycloalkyl)(Heteroaryl);
   C(=O)N(Aryl)(Heteroaryl); OH; OCF₃; O-C₁₋₈-Alkyl; O-C₃₋₁₀-Cycloalkyl; O-Aryl;
   O-Heteroaryl; O-C(=O)-C₁₋₈-Alkyl; O-C(=O)-C₃₋₁₀-Cycloalkyl; O-C(=O)-Aryl; O-C(=O)-Heteroaryl; SH; SCF₃; S-C₁₋₈-Alkyl; S- C₃₋₁₀-Cycloalkyl; S-Aryl; S-Heteroaryl; NH₂; NH-C₁₋₈-Alkyl; NH-C₃₋₁₀-Cycloalkyl; NH-Aryl; NH-Heteroaryl;
   N(C₁₋₈-Alkyl)₂; N(C₃₋₁₀-Cycloalkyl)₂; N(Aryl)₂; N(Heteroaryl)₂; N(C₁₋₈-Alkyl)(C₃₋₁₀-Cycloalkyl); N(C₁₋₈-Alkyl)(Aryl); N(C₁₋₈-Alkyl)(Heteroaryl); N(C₃₋₁₀-Cycloalkyl)(Aryl); N(C₃₋₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl steht;
jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;

### zur Verwendung als Arzneimittel.

Die substituierten Verbindungen der vorstehend genannten allgemeinen Formel (|) und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein solches Arzneimittel enthält wenigstens eine Verbindung der vorstehend angegebenen allgemeinen Formel (I), jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Vorstehend genannte Arzneimittel eignen daher sich insbesondere zur Regulation der vesikulären Glutamat-Transporter 1 und/oder 2 (vGLUT1 und/oder vGLUT2), vorzugsweise zur Hemmung und/oder zur Stimulation der vesikulären Glutamat-Transporter 1 und/oder 2 (vGLUT1 und/oder vGLUT2), d.h. sie üben eine agonistische oder antagonistische Wirkung, vorzugsweise jedoch eine antagonistische Wirkung aus.

Ebenfalls bevorzugt eignen sich derartige Arzneimittel zur Prophylaxe und/oder Behandlung von Störungen oder Krankheiten, die zumindest teilweise durch die vesikulären Glutamat-Transporter 1 und/oder 2 (vGLUT1 und/oder vGLUT2) vermittelt werden.

Insbesondere kann es sich bei dem Arzneimittel um ein Arzneimittel zur Behandlung und/oder Prophylaxe von einer oder mehrerer Erkrankungen handeln, die ausgewählt sind aus der Gruppe umfassend:
Schmerz, akuter Schmerz, chronischer Schmerz, neuropathischer Schmerz,
visceraler Schmerz, inflammatorischer Schmerz, Gelenkschmerz, Hyperalgesie, Allodynie, Kausalgie, Migräne,
neurodegenerative Erkrankungen, Multiple Sklerose, Morbus Alzheimer, Morbus Parkinson, Morbus Huntington, amyotrophe Lateralsklerose,
kognitive Dysfunktionen, kognitive Mangelzustände, Epilepsie,
Störungen der Nahrungsaufnahme, Bulimie, Kachexie, Anorexie, Fettleibigkeit;
Störung des visuellen Systems, Schizophrenie, Diabetes und/oder Depressionen; und/oder weiterhin um ein Arzneimittel
zur Lokalanästhesie; und/oder
zur Hemmung unerwünschter Nebenwirkungen, Übelkeit, Erbrechen, Abhängigkeit, Atemdepression und/oder Obstipation.

Die Ausdrücke "Alkyl" bzw. "C₁₋₁₀-Alkyl", "C₁₋₈-Alkyl", "C₁₋₄-Alkyl" umfassen im Sinne dieser Erfindung acyclische gesättigte oder ungesättigte aliphatische Kohlenwasserstoffreste, d.h. C₁₋₁₀-aliphatische Reste, C₁₋₈-aliphatische Reste und C₁₋₄-aliphatische Reste, die jeweils verzweigt oder unverzweigt sowie unsubstituiert oder ein- oder mehrfach substituiert sein können, mit 1 bis 10 bzw. 1 bis 8 bzw. 1 bis 4 Kohlenstoffatomen, d.h. C₁₋₁₀-Alkanyle, C₂₋₁₀-Alkenyle und C₂₋₁₀-Alkinyle bzw.C₁₋₈-Alkanyle, C₂₋₈-Alkenyle und C₂₋₈-Alkinyle bzw. C₁₋₄-Alkanyle, C₂₋₄-Alkenyle und C₂₋₄-Alkinyle. Dabei weisen Alkenyle mindestens eine C-C-Doppelbindung und Alkinyle mindestens eine C-C-Dreifachbindung auf. Bevorzugt ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, n-Heptyl, n-Octyl, n-Nonyl, n-Decyl, Ethenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Die Ausdrücke "Cycloalkyl" bzw. "C₃₋₁₀-Cycloalkyl" bedeuten für die Zwecke dieser Erfindung cyclische aliphatische (cycloaliphatische) Kohlenwasserstoffe mit insbesondere 3, 4, 5, 6, 7, 8, 9 oder 10 Kohlenstoffatomen, d.h. C₃-₁₀-cycloaliphatische Reste, wobei die Kohlenwasserstoffe gesättigt oder ungesättigt (aber nicht aromatisch), unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, (hetero)cyclischen, aromatischen oder heteroaromatischen Ringsystemen , d.h. mit Cycloalkyl, Heterocyclyl, Aryl oder Heteroaryl kondensiert sein, die wiederum unsubstituiert oder einfach oder mehrfach substituiert sein können. Die Cycloalkyl-Reste können weiterhin einfach oder mehrfach verbrückt sein wie bspw. im Fall von Adamantyl, Bicyclo[2.2.1]heptyl oder Bicyclo[2.2.2]octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl, Cyclononyl, Cyclodecyl, Adamantyl, 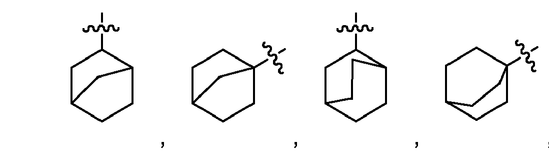 , Cyclopentenyl, Cyclohexenyl, Cycloheptenyl und Cyclooctenyl umfasst.

Die Begriffe "Heterocyclyl" bzw. "Heterocycloalkyl" umfassen aliphatische gesättigte oder ungesättigte (aber nicht aromatische) Cycloalkyle mit insbesondere drei bis zehn, d.h. 3, 4, 5, 6, 7, 8, 9 oder 10 Ringgliedern, in denen mindestens ein, ggf. auch zwei oder drei Kohlenstoffatome durch ein Heteroatom oder eine Heteroatomgruppe jeweils unabhängig voneinander ausgewählt aus der Gruppe bestehend aus O, S, S(=O)₂, N, NH und N(C₁₋₈-Alkyl), vorzugsweise N(CH₃), ersetzt sind, wobei die Ringglieder unsubstituiert oder ein- oder mehrfach substituiert sein können. Heterocyclen sind damit heterocycloaliphatische Reste. Die Bindung des Heterocyclyls an die übergeordnete allgemeine Struktur kann über jedes beliebige und mögliche Ringglied des Heterocyclyl-Restes erfolgen. Die Heterocyclyl-Reste können auch mit weiteren gesättigten, (partiell) ungesättigten (hetero)cyclischen oder aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Bevorzugt sind Heterocyclyl-Reste aus der Gruppe umfassend Azetidinyl, Aziridinyl, Azepanyl, Azocanyl, Diazepanyl, Dithiolanyl, Dihydrochinolinyl, Dihydropyrrolyl, Dioxanyl, Dioxolanyl, Dioxepanyl, Dihydroindenyl Dihydropyridinyl, Dihydrofuranyl, Dihydroisochinolinyl, Dihydroindolinyl, Dihydroisoindolyl, lmidazolidinyl, Isoxazolidinyl, Morpholinyl, Oxiranyl, Oxetanyl, Pyrrolidinyl, Piperazinyl, 4-Methylpiperazinyl, Piperidinyl, Pyrazolidinyl, Pyranyl, Tetrahydropyrrolyl, Tetrahydropyranyl, Tetrahydrochinolinyl, Tetrahydroisochinolinyl, Tetrahydroindolinyl, Tetrahydrofuranyl, Tetrahydropyridinyl, Tetrahydrothiophenyl, Tetrahydro-pyridoindolyl, Tetrahydronaphthyl, Tetrahydrocarbolinyl, Tetrahydroisoxazolo-pyridinyl, Thiazolidinyl und Thiomorphol inyl.

Der Begriff "Aryl" bedeutet im Sinne dieser Erfindung aromatische Kohlenwasserstoffe mit bis zu 14 Ringgliedern, insbesondere Phenyle und Naphthyle. Jeder ArylRest kann unsubstituiert oder einfach oder mehrfach substituiert vorliegen, wobei die Aryl-Substituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl-Reste können auch mit weiteren gesättigten, (partiell) ungesättigten, (hetero)cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Beispiele für kondensierte Aryl-Reste sind Benzodioxolanyl und Benzodioxanyl. Bevorzugt ist Aryl aus der Gruppe ausgewählt, die Phenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5- oder 6-gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N und O und der Heteroaryl-Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Die Bindung an die übergeordnete allgemeine Struktur kann über jedes beliebige und mögliche Ringglied des Heteroaryl-Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, (hetero)cyclischen oder aromatischen oder heteroaromatischen Ringen gebildet werden kann, d.h. mit Cycloalkyl, Heterocyclyl, Aryl oder Heteroaryl, die wiederum unsubstituiert oder einfach oder mehrfach substituiert sein können. Es ist bevorzugt, dass der Heteroaryl-Rest ausgewählt ist aus der Gruppe, die Benzofuranyl, Benzoimidazolyl, Benzothienyl, Benzothiadiazolyl, Benzothiazolyl, Benzotriazolyl, Benzooxazolyl, Benzooxadiazolyl, Chinazolinyl, Chinoxalinyl, Carbazolyl, Chinolinyl, Dibenzofuranyl, Dibenzothienyl, Furyl (Furanyl), Imidazolyl, lmidazothiazolyl, Indazolyl, Indolizinyl, Indolyl, Isochinolinyl, Isoxazoyl, Isothiazolyl, Indolyl, Naphthyridinyl, Oxazolyl, Oxadiazolyl, Phenazinyl, Phenothiazinyl, Phtalazinyl, Pyrazolyl, Pyridyl (2-Pyridyl, 3-Pyridyl, 4-Pyridyl), Pyrrolyl, Pyridazinyl, Pyrimidinyl, Pyrazinyl, Purinyl, Phenazinyl, Tetrazol, Thienyl (Thiophenyl), Triazolyl, Tetrazolyl, Thiazolyl, Thiadiazolyl oder Triazinyl umfasst. Besonders bevorzugt sind Tetrazol, Pyridyl und Thienyl.

Die Ausdrücke "über C₁₋₄-Alkyl oder C₁₋₈-Alkyl verbrücktes Aryl, Heteroaryl, Heterocyclyl oder Cycloalkyl" bedeuten im Sinne der Erfindung, dass C₁₋₄-Alkyl oder C₁₋₈-Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cycloalkyl-Rest über eine C₁₋₄-Alkyl- oder eine C₁₋₈-Alkyl-Gruppe an die jeweilige übergeordnete allgemeine Struktur gebunden ist. Die Alkylkette der Alkyl-Gruppe kann in allen Fällen verzweigt oder unverzweigt, unsubstituiert oder einfach oder mehrfach substituiert sein. Die Alkylkette der Alkyl-Gruppe kann ferner in allen Fällen gesättigt oder ungesättigt sein, d.h. kann eine Alkylengruppe, d.h. eine C₁₋₄-Alkylengruppe oder eine C₁₋₈-Alkylengruppe, eine Alkenylengruppe, d.h. eine C₂₋₄-Alkenylengruppe oder eine C₂₋₈-Alkenylengruppe, oder eine Alkinylengruppe, d.h. eine C₂₋₄-Alkinylengruppe oder eine C₂₋₈-Alkinylengruppe sein. Vorzugsweise ist C₁₋₄-Alkyl ausgewählt aus der Gruppe umfassend -CH₂-, -CH₂-CH₂-, -CH(CH₃)-, -CH₂-CH₂-CH₂-, -CH(CH₃)-CH₂-, -CH(CH₂CH₃)-**,** -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -C=C-, -C≡C-CH₂-, -C=C-CH₂-CH₂-, -C≡C-CH(CH₃)-, -CH₂-C≡C-CH₂- und -C≡C-C≡C- und C₁₋₈-Alkyl ausgewählt aus der Gruppe umfassend -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH₂-(CH₂)₃-CH₂-, -CH(CH₃)-CH₂-CH₂-CH₂-, -CH₂-CH(CH₃)-CH₂-CH₂-, -CH(CH₃)-CH₂-CH(CH₃)-, -CH(CH₃)-CH(CH₃)-CH₂-, -C(CH₃)₂-CH₂-CH₂-, -CH₂-C(CH₃)₂-CH₂-, -CH(CH₂CH₃)-CH₂-CH₂-, -CH₂-CH(CH₂CH₃)-CH₂-, -C(CH₃)₂-CH(CH₃)-, -CH(CH₂CH₃)-CH(CH₃)-, -C(CH₃)(CH₂CH₃)-CH₂-, -CH(CH₂CH₂CH₃)-CH₂-, -C(CH₂CH₂CH₃)-CH₂-, -CH(CH₂CH₂CH₂CH₃)-, -C(CH₃)(CH₂CH₂CH₃)-, -C(CH₂CH₃)₂-, -CH₂-(CH₂)₄-CH₂-, -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -CH=CH-CH₂-CH₂-CH₂-, -CH₂-CH=CH₂-CH₂-CH₂-, -CH=CH=CH-CH₂-CH₂-, -CH=CH₂-CH-CH=CH₂-, -C=C-, -C≡C-CH₂-, -C≡C-CH₂-CH₂-, -C≡C-CH(CH₃)-, -CH₂-C≡-CH₂-, -C≡C-C≡C-, -C-C(CH₃)₂-, -C≡C-CH₂-CH₂-CH₂-, -CH₂-C≡C-CH₂-CH₂-, -C≡C-C≡C-CH₂- und -C≡C-CH₂-C≡C-.

Im Zusammenhang mit "Alkyl", "Heterocyclyl" und "Cycloalkyl" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome mit Substituenten jeweils unabhängig voneinander ausgewählt aus der Gruppe bestehend aus F; Cl; Br; l; NO₂; CN; =O; CF₃; C₁₋₈-Alkyl, Aryl; Heteroaryl; C₃₋₁₀-Cycloalkyl; Heterocyclyl; C(=O)OH; C(=O)O-C-₁₋₈-Alkyl; C(=O)O-C₃-₁₀-Cycloalkyl; C(=O)O-Aryl; C(=O)O-Heteroaryl; C(=O)NH₂; C(=0)NH-C₁₋₈-Alkyl; C(=O)NH-C₃₋₁₀-Cycloalkyl; C(=O)NH-Aryl; C(=O)NH-Heteroaryl; C(=O)N(C₁₋₈-Alkyl)₂; C(=O)N(C₃₋₁₀-Cycloalkyl)₂; C(=O)N(Aryl)₂; C(=O)N(Heteroaryl)₂; C(=O)N(C₁₋₈-Alkyl)(C₃-₁₀-Cydoalkyl); C(=O)N(C₁₋₈-Alkyl)(Aryl); C(=O)N(C₁₋₈-Alkyl)(Heteroaryl); C(=O)N(C₃₋₁₀-Cycloalkyl)(Aryl); C(=O)N(C₃₋₁₀-Cycloalkyl)(Heteroaryl); C(=O)N(Aryl)(Heteroaryl); OH; OCF₃; O-C₁₋₈-Alkyl; O-C₃₋₁₀-Cycloalkyl; O-Aryl; O-Heteroaryl; O-C(=O)-C₁₋₈-Alkyl; O-C(=O)-C₃₋₁₀-Cycloalkyl; O-C(=O)-Aryl; O-C(=O)-Heteroaryl; SH; SCF₃; S-C₁₋₈-Alkyl; S- C₃₋₁₀-Cycloalkyl; S-Aryl; S-Heteroaryl; NH₂; NH-C₁₋₈-Alkyl; NH-C₃₋₁₀-Cycloalkyl; NH-Aryl; NH-Heteroaryl; N(C₁₋₈-Alkyl)₂; N(C₃₋₁₀-Cycloalkyl)_{2;} N(Aryl)₂; N(Heteroaryl)₂; N(C₁₋₈-Alkyl)(C₃₋₁₀-Cycloalkyl); N(C₁₋₈-Alkyl)(Aryl); N(C₁₋₈-Alkyl)(Heteroaryl); N(C₃₋₁₀-Cycloalkyl)(Aryl); N(C₃₋₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl; wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C-Atom wie im Falle von CF₃ oder CH₂CF₃ oder an verschiedenen Stellen wie im Falle von CH(OH)-CH=CH-CHCl₂. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl-", "Heterocyclyl-" und "Cycloalkyl-" Substituenten sind ausgewählt aus der Gruppe bestehend aus F; Cl; Br; l; NO₂; CN; =O; CF₃, Cₗ₋₈-Alkyl, Phenyl; Naphthyl; Pyridyl; Thienyl; C₃₋₁₀-Cycloalkyl; Heterocyclyl; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=O)N(C₁₋₈-Alkyl)₂; C(=O)N(C₁₋₈-Alkyl)(Phenyl); OH; OCF₃; O-C₁-₈₋Alkyl; O-C₃₋₁₀-Cycloalkyl, O-Phenyl; O-C(=O)-C₁₋₈-Alkyl; O-C(=O)-C₃₋₁₀-Cycloalkyl; O-C(=O)-Phenyl; SH; SCF₃; S-C₁₋₈-Alkyl; NH₂; NH-C₁₋₈-Alkyl; NH-C₃₋₁₀-Cycloalkyl; NH-Phenyl; N(C₁₋₈-Alkyl)₂;N(C₁₋₈-Alkyl)(Phenyl) und NH(C=O)-C₁₋₈-Alkyl.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems mit Substituenten jeweils unabhängig voneinander ausgewählt aus der Gruppe bestehend aus F; Cl; Br; l; NO₂; CN; CF₃; C₁₋₈-Alkyl; Aryl; Heteroaryl; C₃₋₁₀-Cycloalkyl; Heterocyclyl; C(=O)OH; C(=O)O-C₁₋₈-Alkyl; C(=O)O-C₃₋₁₀-Cycloalkyl; C(=O)O-Aryl; C(=O)O-Heteroaryl; C(=O)NH₂; C(=O)NH-C₁₋₈-Alkyl; C(=O)NH-C₃₋₁₀-Cycloalkyl; C(=O)NH-Aryl; C(=O)NH-Heteroaryl; C(=O)N(C₁₋₈-Alkyl)₂; C(=O)N(C₃-₁₀-Cycloalkyl)₂; C(=O)N(Aryl)₂; C(=O)N(Heteroaryl)₂; C(=O)N(C₁₋₈-Alkyl)(C₃₋₁₀-Cycloalkyl); C(=O)N(C₁₋₈-Alkyl)(Aryl); C(=O)N(C₁₋₈-Alkyl)(Heteroaryl); C(=O)N(C₃₋₁₀-Cycloalkyl)(Aryl); C(=O)N(C₃₋₁₀-Cycloalkyl)(Heteroaryl); C(=O)N(Aryl)(Heteroaryl); OH; OCF₃; O-C₁₋₈-Alkyl; O-C₃₋₁₀-Cycloalkyl; O-Aryl; O-Heteroaryl; O-C(=O)-C₁₋₈-Alkyl; O-C(=O)-C₃₋₁₀-Cycloalkyl; O-C(=O)-Aryl; O-C(=O)-Heteroaryl; SH; SCF₃; S-C₁₋₈-Alkyl ; S- C₃₋₁₀-Cycloalkyl, S-Aryl; S-Heteroaryl; NH₂; NH-C₁₋₈-Alkyl; NH-C₃₋₁₀-Cycloalkyl; NH-Aryl; NH-Heteroaryl; N(C₁₋₈-Alkyl)₂; N(C₃₋₁₀-Cycloalkyl)₂; N(Aryl)₂; N(Heteroaryl)₂; N(C₁₋₈-Alkyl)(C₃₋₁₀-Cycloalkyl); N(C₁₋₈-Alkyl)(Aryl); N(C₁₋₈-Alkyl)(Heteroaryl); N(C₃₋₁₀-Cycloalkyl)(Aryl); N(C₃₋₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl; an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl-" und "Heteroaryl-" Substituenten sind ausgewählt aus der Gruppe bestehend aus F; Cl; Br; l; NO₂; CN; =O; CF₃; C₁₋₈-Alkyl; Phenyl; Naphthyl; Pyridyl; Thienyl; C₃₋₁₀-Cycloalkyl; Heterocyclyl; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=O)N(C₁₋₈-Alkyl)_{2;} C(=O)N(C₁₋₈-Alkyl)(Phenyl); OH; OCF₃; O-C₁₋₈-Alkyl; O-C₃₋₁₀-Cycloalkyl; O-Phenyl; O-C(=O)-C₁₋₈-Alkyl; O-C(=O)-C₃₋₁₀-Cycloalkyl; O-C(=O)-Phenyl; SH; SCF₃; S-C₁₋₈-Alkyl; NH₂; NH-C₁₋₈-Alkyl; NH-C₃₋₁₀-Cycloalkyl; NH-Phenyl; N(C₁₋₈-Alkyl)_{2;} N(C₁₋₈-Alkyl)(Phenyl) und NH(C=O)-C₁₋₈-Alkyl.

Die Verbindungen der allgemeinen Formel (l) sind durch Substituenten definiert, beispielsweise durch R¹, R² und R³ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise R² = Aryl (Substituent der 1. Generation), so kann Aryl seinerseits substituiert sein, z.B. mit C₁₋₈-Alkyl (Substituent der 2. Generation). Es ergibt sich daraus die funktionelle Gruppe Aryl-C₁₋₈-Alkyl. C₁₋₈-Alkyl kann dann seinerseits erneut substituiert sein, z.B. mit Cl (Substituent der 3. Generation). Es ergibt sich daraus dann insgesamt die funktionelle Gruppe Aryl-C₁₋₈-Alkyl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2.

Generation nicht erneut substituiert sein, d.h. es gibt dann bereits keine Substituenten der 3. Generation. Mit anderen Worten können in dieser Ausführungsform bspw. im Fall der allgemeinen Formel (l) die funktionellen Gruppen für R¹ bis R¹⁰ jeweils ggf. substituiert sein, die jeweiligen Substituenten können dann ihrerseits jedoch nicht erneut substituiert sein.

Im Rahmen der vorliegenden Erfindung bezeichnet das in Formeln verwendete Symbol 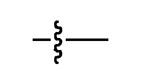 eine Verknüpfung eines entsprechenden Restes an die jeweilige übergeordnete allgemeine Struktur.

Wenn ein Rest innerhalb der Definition eines anderen Restes des Moleküls mehrfach genannt ist, wie z.B. der Rest R⁶ als OR⁶ oder als NR⁶R⁷ innerhalb der Definition von R¹, dann sind die Bedeutungen dieses Restes jeweils unabhängig voneinander: beispielsweise kann R⁶ in OR⁶ O-C₁₋₁₀-Alkyl bedeuten und in NR⁶R⁷ dagegen für Aryl stehen, so dass sich die Gruppe N(Aryl)R⁷ ergibt.

Unter dem Begriff des physiologisch verträglichen Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für Säuren, die zur Bildung entsprechender Salze geeignet sind, sind Salzsäure, Bromwasserstoffsäure, Schwefelsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure, Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetierverträglich sind. Besonders bevorzugt sind die Salze der Alkali- und Erdalkalimetalle aber auch Ammoniumsalze [NH_{X}R₄-_{X}]⁺, worin x = 0, 1, 2, 3 oder 4 ist und R für einen verzweigten oder unverzweigten, gesättigten oder ungesättigten C₁₋₁₀-Alkyl-Rest, unsubstituiert oder einfach oder mehrfach substituiert oder für einen C₃₋₁₀-Cycloalkyl-Rest, gesättigt oder ungesättigt, unsubstituiert oder einfach oder mehrfach substituiert steht oder zwei der Reste R gemeinsam mit dem Stickstoffatom ein Heterocyclyl, gesättigt oder ungesättigt, unsubstituiert oder einfach oder mehrfach substituiert, vorzugsweise ein Pyrrolidinyl, Piperidinyl oder Morpholinyl bilden. Ganz besonders bevorzugt sind (Mono-) oder (Di-) Lithium-, (Mono-) oder (Di-) Natrium-, (Mono-) oder (Di-) Kalium-, Magnesium- oder Calcium-Salze.

Steht X in der allgemeinen Formel (I) für -NHC(=O)-, so ist die Carbonylgruppe an den Dioxoisoindolin-Grundkörper gebunden. Steht X in der allgemeinen Formel (I) für -C(=O)NH-, so ist dementsprechend die NH-Funktion an den Dioxoisoindolin-Grundkörper gebunden.

Wenn im Folgenden bevorzugte Ausführungsformen der Verbindungen der allgemeinen Formel (I) angeführt werden, so betrifft dies bevorzugte Ausführungsformen der Verbindungen der allgemeinen Formel (I) zur Verwendung als Arzneimittel.

Bevorzugte Ausführungsformen der Verbindungen der allgemeinen Formel (I) haben die allgemeinen Formeln (la), (lb) und (Ic):

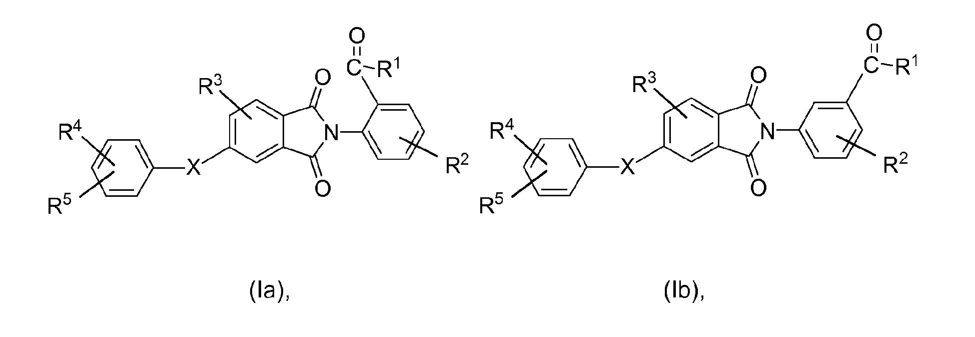

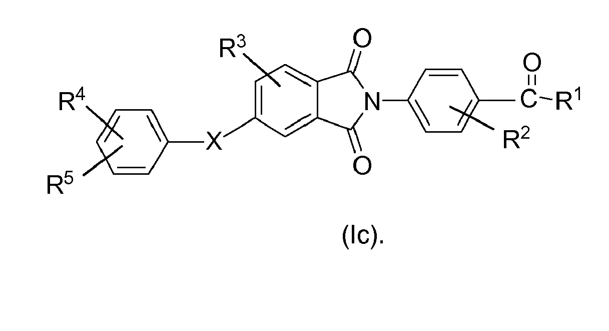

Eine weitere Ausführungsform der vorliegenden Erfindung betrifft wenigstens eine substituierte Verbindung (l), wie sie vorstehend beschrieben wurde, wobei X für -NH(C=O)- oder für 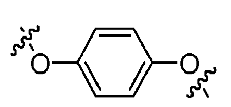 steht, zur Verwendung als Arzneimittel.

Eine weitere Ausführungsform der vorliegenden Erfindung betrifft wenigstens eine substituierte Verbindung (l), wie sie vorstehend beschrieben wurde, wobei

R¹ für OR⁶; oder NR⁶R⁷ steht,
wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl, NH₂, F, Cl, Br und l_{;} C₃₋₁₀₋Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und l_{;} Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und l; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH2, F, Cl, Br und l, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und l, wobei die Alkylkette unsubstituiert ist und jeweils verzweigt oder unverzweigt, gesättigt oder ungesättigt sein kann; stehen,

zur Verwendung als Arzneimittel.

Eine weitere Ausführungsform der vorliegenden Erfindung betrifft wenigstens eine substituierte Verbindung (l), wie sie vorstehend beschrieben wurde, wobei

R¹ für OR⁶ oder NR⁶R⁷ steht,
wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl und NH₂; Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; über C₁₋₈-Alkyl verbrücktes Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂, wobei die Alkylkette unsubstituiert ist und jeweils verzweigt oder unverzweigt, gesättigt oder ungesättigt; oder über C₁-₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C-₁-₄-Alkyl, NH₂ und F, wobei die Alkylkette unsubstituiert ist und jeweils verzweigt oder unverzweigt, gesättigt oder ungesättigt sein kann, stehen,

zur Verwendung als Arzneimittel.

Wenn R¹ für NR⁶R⁷ steht, steht der Rest R⁷ in einer besonders bevorzugten Ausführungsform der Verbindungen der allgemeinen Formel (l) für H und R⁶ steht für C₁-₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁-₄-Alkyl und NH₂; Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁-₄-Alkyl und NH₂; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁-₄-Alkyl und NH₂; über C₁-₈-Alkyl verbrücktes Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁-₄-Alkyl, NH₂, wobei die Alkylkette unsubstituiert ist und jeweils verzweigt oder unverzweigt, gesättigt oder ungesättigt sein kann; oder über C₁-₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁-₄-Alkyl, NH₂ und F, wobei die Alkylkette unsubstituiert ist und jeweils verzweigt oder unverzweigt, gesättigt oder ungesättigt sein kann.

Eine weitere Ausführungsform der vorliegenden Erfindung betrifft wenigstens eine substituierte Verbindung (l), wie sie vorstehend beschrieben wurde, wobei

R² und R³ jeweils unabhängig voneinander für H; F; Cl; Br; I; OR⁸; C(=O)OR⁸; CF₃; NR⁸R⁹; C₁-₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I_{;} C₃-₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁-₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I_{;} über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I., wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH2, F, Cl, Br und I_{;} wobei die Alkylkette unsubstituiert ist und jeweils verzweigt oder unverzweigt, gesättigt oder ungesättigt sein kann; stehen,

wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈Alkyl verbrücktes C₃-₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I_{;}, wobei die Alkylkette unsubstituiert ist und jeweils verzweigt oder unverzweigt, gesättigt oder ungesättigt sein kann; stehen,

zur Verwendung als Arzneimittel.

Eine weitere Ausführungsform der vorliegenden Erfindung betrifft wenigstens eine substituierte Verbindung (l), wie sie vorstehend beschrieben wurde, wobei

R² und R³ jeweils unabhängig voneinander für H; F; Cl; Br; I; OH; C(=O)OH; CF₃; C₁₋₁₀-Alkyl, gesättigt oder ungesättigt, verzweigt oder unverzweigt, unsubstituiert; Phenyl, unsubstituiert; oder über C₁₋₈-Alkyl-verbrücktes Phenyl, jeweils unsubstituiert, stehen, zur Verwendung als Arzneimittel.

### Vorzugsweise stehen

R² und R³ jeweils unabhängig voneinander für H; F; Cl; Br; I; OH; C(=O)OH; CF₃; C₁₋₁₀-Alkyl, gesättigt oder ungesättigt, verzweigt oder unverzweigt, unsubstituiert; Phenyl, unsubstituiert; oder über C₁₋₈-Alkyl-verbrücktes Phenyl, jeweils unsubstituiert, stehen.

Besonders bevorzugt steht R³ für H und R² bedeutet H; F; Cl; Br; I; OH; C(=O)OH; CF₃; oder C₁₋₁₀-Alkyl, gesättigt oder ungesättigt, verzweigt oder unverzweigt, unsubstituiert.

Eine weitere Ausführungsform der vorliegenden Erfindung betrifft wenigstens eine substituierte Verbindung (l), wie sie vorstehend beschrieben wurde, wobei

R⁴ und R⁵ jeweils unabhängig voneinander für H; F; Cl; Br; I; OR⁸; C(=O)OR⁸; CF₃; NR⁸R⁹; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁-₄-Alkyl , NH₂, F, Cl, Br und I; Aryl oder Heteroaryl, ; jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁-₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,

wobei R⁸ und R⁹ jeweils unabhängig voneinander für H; C₁₋₁₀-AlkyL gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, 0-C₁₋₄-Alkyl, NH₂, F, Cl, Br und I, über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; stehen; oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,

wobei R¹⁰ für C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und l; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette jeweils unsubstituiert ist und verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und l, wobei die Alkylkette unsubstituiert ist und jeweils verzweigt oder unverzweigt, gesättigt oder ungesättigt sein kann; steht,

zur Verwendung als Arzneimittel.

Eine weitere Ausführungsform der vorliegenden Erfindung betrifft wenigstens eine substituierte Verbindung (l), wie sie vorstehend beschrieben wurde, wobei

R⁴ und R⁵ jeweils unabhängig voneinander für H; F; Cl; Br; I; OH; C(=O)OH; CF₃; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und l; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I: oder über C₁₋₃-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH2, F, Cl, Br und l; wobei die Alkylkette unsubstituiert ist und jeweils verzweigt oder unverzweigt, gesättigt oder ungesättigt sein kann; stehen,

oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,

wobei R¹⁰ für C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁.. ₄-Alkyl, NH₂, F, Cl, Br und I, Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I: oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; steht,

zur Verwendung als Arzneimittel.

Eine weitere Ausführungsform der vorliegenden Erfindung betrifft wenigstens eine substituierte Verbindung (l), wie sie vorstehend beschrieben wurde, wobei
X für -NH(C=O)- oder für 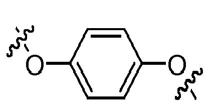 steht,
R¹ für OR⁶ oder NR⁶R⁷ steht,

wobei R⁷ für H steht und R⁶ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl und NH₂; Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; überC₁₋₈-Alkyl verbrücktes Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂, wobei die Alkylkette unsubstituiert ist und jeweils verzweigt oder unverzweigt, gesättigt oder ungesättigt sein kann; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁-₄-Alkyl, C(=O)OH, OH, O-C₁₋₄-Alkyl, NH₂ und F, wobei die Alkylkette unsubstituiert ist und jeweils verzweigt oder unverzweigt, gesättigt oder ungesättigt sein kann; stehen;

R² und R³ jeweils unabhängig voneinander für H; F; Cl; Br; I; OH; C(=O)OH; CF₃; C₁₋₁₀-Alkyl, gesättigt oder ungesättigt, verzweigt oder unverzweigt, unsubstituiert; Phenyl, unsubstituiert; oder über C₁₋₈-Alkyl-verbrücktes Phenyl, jeweils unsubstituiert, stehen,

R⁴ und R⁵ jeweils unabhängig voneinander für H; F; Cl; Br; I; OH; C(=O)OH; CF₃; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,

oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,

wobei R¹⁰ für C₁₋₁₀-Alkyl, gesättigt oder ungesättigt, verzweigt oder unverzweigt, unsubstituiert oder einfach oder mehrfach

substituiert mit einem oder mehreren Substituenten jeweils unabhängig voneinander ausgewählt aus der Gruppe bestehend aus, C(=O)OH, OH, O-C₁₋₄-Alkyl, NH2, F, Cl, Br und I_{;} Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH2, F, Cl, Br und I_{;} wobei die Alkylkette unsubstituiert ist und jeweils verzweigt oder unverzweigt, gesättigt oder ungesättigt sein kann_{;} steht,

zur Verwendung als Arzneimittel.

Eine weitere Ausführungsform der vorliegenden Erfindung betrifft wenigstens eine substituierte Verbindung (l), wie sie vorstehend beschrieben wurde, ausgewählt aus der Gruppe
1 2,2'-(5,5'-(1,4-Phenylenbis(oxy))bis(1,3-dioxoisoindolin-5,2-diyl))dibenzoesäure;
2 2-(4-(5-(2-Carboxy-4-hydroxyphenylcarbamoyl)-1,3-dioxoisoindolin-2-yl)benzamido)-5-hydroxybenzoesäure;
3 2-(3-(5-(2-Carboxyphenylcarbamoyl)-1,3-dioxoisoindolin-2-yl)benzamido)-benzoesäure und
4 2-(4-(5-(2-Carboxylato-4-hydroxyphenylcarbamoyl)-1,3-dioxoisoindolin-2-yl)-benzamido)-5-hydroxybenzoesäure,

jeweils in Form der freien Verbindungen und/oder in Form der Salze physiologisch verträglicher Säuren oder Basen,

### zur Verwendung als Arzneimittel.

Ein weiterer Gegenstand der vorliegenden Erfindung ist wenigstens eine substituierte Verbindung (l,) wie sie vorstehend beschrieben wurde,

jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;

zur Behandlung und/oder Prophylaxe von einer oder mehreren Erkrankungen ausgewählt aus der Gruppe umfassend:
Schmerz, akuter Schmerz, chronischer Schmerz, neuropathischer Schmerz,
visceraler Schmerz, inflammatorischer Schmerz, Gelenkschmerz, Hyperalgesie, Allodynie, Kausalgie, Migräne,
neurodegenerative Erkrankungen, Multiple Sklerose, Morbus Alzheimer,
Morbus Parkinson, Morbus Huntington, amyotrophe Lateralsklerose,
kognitive Dysfunktionen, kognitive Mangelzustände, Epilepsie,
Störungen der Nahrungsaufnahme, Bulimie, Kachexie, Anorexie,
Fettleibigkeit;
Störung des visuellen Systems, Schizophrenie, Diabetes und/oder Depressionen; und/oder
zur Lokalanästhesie; und/oder
zur Hemmung unerwünschter Nebenwirkungen, übelkeit, Erbrechen, Abhängigkeit, Atemdepression und/oder Obstipation.

Ein weiterer Gegenstand der vorliegenden Erfindung ist die Verwendung wenigstens einer substituierten Verbindung der allgemeinen Formel (l), wie sie vorstehend beschrieben wurde, in der X, R¹, R², R³, R⁴, R⁵, R⁶ R⁷, R⁸, R⁹ und R¹⁰ die Bedeutung gemäß der vorstehenden Beschreibung haben,

jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;

zur Herstellung eines Arzneimittels zur Behandlung und/oder Prophylaxe von einer oder mehrerer Erkrankungen ausgewählt aus der Gruppe umfassend:
Schmerz, akuter Schmerz, chronischer Schmerz, neuropathischer Schmerz, visceraler Schmerz, inflammatorischer Schmerz, Gelenkschmerz, Hyperalgesie, Allodynie, Kausalgie, Migräne,
neurodegenerative Erkrankungen, Multiple Sklerose, Morbus Alzheimer, Morbus Parkinson, Morbus Huntington, amyotrophe Lateralsklerose,
kognitive Dysfunktionen, kognitive Mangelzuständen, Epilepsie,
Störungen der Nahrungsaufnahme, Bulimie, Kachexie, Anorexie, Fettleibigkeit;
Störung des visuellen Systems, Schizophrenie, Diabetes und/oder Depressionen; und/oder

zur Lokalanästhesie; und/oder

zur Hemmung unerwünschter Nebenwirkungen, Übelkeit, Erbrechen, Abhängigkeit, Atemdepression und/oder Obstipation.

Ein weiterer Gegenstand der vorliegenden Erfindung ist ein Arzneimittel, enthaltend wenigstens eine substituierte Verbindung der allgemeinen Formel (I), wie sie vorstehend beschrieben wurde, in der X, R¹, R², R³, R⁴, R⁵, R⁶ R⁷, R⁸, R⁹ und R¹⁰ die Bedeutung gemäß der vorstehenden Beschreibung haben.

Das Arzneimittel eignet sich zur Verabreichung an Erwachsene und Kinder einschließlich Kleinkindern und Säuglingen und kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpresst, in Kapseln abgefüllt oder in einer Flüssigkeit suspendiert, vorliegen und als solche auch verabreicht werden.

Neben wenigstens einer substituierten Verbindung der vorstehend angegebenen allgemeinen Formel (I), ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem Arzneimittel zum Einsatz kommenden substituierten Verbindungen der allgemeinen Formel (I) in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige substituierte Verbindung der allgemeinen Formel (I) auch verzögert freisetzen.

Die Herstellung der Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen substituierten Verbindungen der vorstehend angegebenen allgemeinen Formel (I) kann variieren und ist beispielsweise abhängig vom Gewicht oder Alter des Patienten sowie von der Applikationsart, der Indikation und dem Schweregrad der Erkrankung. Üblicherweise werden 0.001 bis 100 mg/kg, vorzugsweise 0.05 bis 75 mg/kg, besonders bevorzugt 0.05 bis 50 mg/kg, Körpergewicht des Patienten wenigstens einer solchen Verbindung der allgemeinen Formel (I) appliziert.

Die Verbindungen der vorstehend angegebenen Formel (I) oder entsprechende Ausgangsstoffe, die zur Synthese dieser Verbindungen eingesetzt werden können, sind jeweils am Markt käuflich erhältlich und/oder können auch nach üblichen, dem Fachmann bekannten Verfahren hergestellt werden.

Die Umsetzungen der Ausgangsstoffe können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre, durchgeführt werden.

Die substituierten Verbindungen der vorstehend genannten allgemeinen Formel (I) sowie ggf. entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen substituierten Verbindungen der vorstehend genannten allgemeinen Formel (I) sowie entsprechender Stereoisomere können beispielsweise durch Umsetzung mit einer anorganischen oder organischen Säure, vorzugsweise mit Salzsäure, Bromwasserstoffsäure, Schwefelsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und/oder Asparaginsäure, in die entsprechenden Salze, vorzugsweise physiologisch verträglichen Salze, überführt werden. Die freien Basen der jeweiligen substituierten Verbindungen der vorstehend genannten allgemeinen Formel (I) und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Verbindungen der vorstehend genannten allgemeinen Formel (I) und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄-ₓ]⁺, worin x = 0, 1, 2, 3 oder 4 ist und R für einen verzweigten oder unverzweigten, gesättigten oder ungesättigten C₁₋₁₀-Alkyl-Rest, unsubstituiert oder einfach oder mehrfach substituiert oder für einen C₃-₁₀-Cycloalkyl-Rest, gesättigt oder ungesättigt, unsubstituiert oder einfach oder mehrfach substituiert steht oder zwei der Reste R gemeinsam mit dem Stickstoffatom ein Heterocyclyl, gesättigt oder ungesättigt, unsubstituiert oder einfach oder mehrfach substituiert, vorzugsweise ein Pyrrolidinyl, Piperidinyl oder Morpholinyl bilden, genannt.

Die substituierten Verbindungen der vorstehend genannten allgemeinen Formel (I) und ggf. entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die substituierten Verbindungen der vorstehend genannten allgemeinen Formel (I) nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

### Pharmakologische Methoden

### I. Funktionelle Untersuchung an den vGLUT1- bzw. vGLUT2-Transportern

Die agonistische bzw. antagonistische Wirkung der zu untersuchenden Verbindungen am vGLUT1- bzw. vGLUT2-Transporter der Spezies Ratte kann mit folgendem Assay bestimmt werden.

### Glutamat-Uptake in Rattenvesikel ('rat crude synaptic vesicles')

Die Präparation von Vesikeln wurde aus Rattengehirn-homogenaten (männlichen Ratten, Stamm Wistar SPF, 150-250 g Gewicht) durchgeführt gemäß Roseth et al. (1995) J. Neurochem. 65: 96-103, der sich auf Whittaker et al. (1964) Biochem J. 90, 293-303 und Fykse und Fonnum (1988) J. Neurochem. 50: 1237-1242 bezieht.

Hierzu wurden frisch entnommene Rattengehirne in 0,32 M Saccharose, 10 mM TRIS-maleat, 1 mM EGTA, pH 7,4 (10 ml/g Frischgewicht) mit einem Potter-Homogenisator (Fa. Braun/Melsungen; 5 Kolbenhübe bei 800 Upm) unter Eiskühlung homogenisiert und anschließend für 10 Minuten bei 3.000 g und 4°C zentrifugiert. Der resultierende Überstand wurde bei 20.000 g für 30 Minuten bei 4°C zentrifugiert. Das entstandene Pellet wurde dann im Verhältnis 1:3 mit 10 mM TRIS-maleate, 0,1 mM EGTA, pH 7,4 osmotisch geschockt und die Suspension bei 17000g für 30 min (4°C) zentrifugiert. Die im Überstand verbliebenen Vesikel wurden nun auf einen Dichtegradienten aus 0,6 M Saccharose in 10 mM TRIS-maleat, 1 mM EGTA, pH 7.4 und 0.4 M Saccharose in 10 mM TRIS-maleat, 1 mM EGTA, pH 7.4 geschichtet. Eine Ultrazentrifugation der Gradienten im Ausschwingrotor bei 65000g für 2h (4°C) schloss sich an. Die Vesikel-Fraktion wurde von der 0.4 M Saccharose-Bande isoliert und im Uptake-Versuch eingesetzt.

Die Substratlösung für den Uptake von [³H]-Glutamat bestand aus ATP (neutralisiert mit Trisma base; 2 mM im Endansatz), Glutamat (0.5 mM im Endansatz) und ³H-Glutamat (0.5 µCi im Endansatz). In 14 ml-Reagiergefäßen (Sarstedt, Artikel-Nr.: 798438) wurden jeweils 0,3 ml Gesamtvolumina (n=2/Probe) pipettiert in der folgenden Reihenfolge: 1.) 165 µl Inkubationspuffer (110 mM Kaliumtartrat, 10 mM TRIS-maleat, pH 7,4, 4 mM MgCl₂), 2.) 10 µl Testsubstanz oder Vehikelkontrolle oder blank, 3.) 100 µl synaptische Vesikel. Es schloss sich eine Vorinkubation von 15 min bei 30°C an (nur blank-Ansätze befanden sich hierbei auf Eis). Danach wurde der Uptake durch Zugabe von 25 µl der oben beschriebenen Substratlösung gestartet. Die Reaktion wurde nach 5 min durch Zugabe von 7,0 ml eiskalter 150 mM KCI gestoppt. Anschließend wurden die Proben mit Hilfe des Brandel Cell Harvesters Typ M-24R über einen Membranfilter der Porengröße 0,8 µm abgesaugt und zweimal mit eiskalter 150 mM KCI nachgewaschen. Die Messung erfolgte am beta-Counter nach 15 h in 10 ml Szintillatorflüssigkeit (Filter Count).

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele

Die Verbindungen der allgemeinen Formel (I) oder Ausgangsstoffe zu ihrer Darstellung sowie weitere Chemikalien und Lösungsmittel sind kommerziell verfügbar (Anbieter wie bspw. Acros, Avocado, Aldrich, Bachem, Fluka, Lancaster, Maybridge, Merck, Sigma, ChemBridge Corporation, I.F. Lab TCI, Oakwood etc. können beispielsweise in der SymyxⓇ Available Chemicals Database der Firma MDL, San Ramon, US recherchiert werden) oder deren Synthese ist in der Fachliteratur bereits genau beschrieben (Experimentelle Vorschriften können beispielweise in der Reaxys® Datenbank der Firma Elsevier, Amsterdam, NL recherchiert werden) oder können nach den üblichen dem Fachmann bekannten Methoden hergestellt werden.

Die Ausbeuten der wie nachstehend beschrieben hergestellten Verbindungen wurden nicht optimiert.

Alle Temperaturen sind unkorrigiert.

Die analytische Charakterisierung aller Zwischenprodukte und Beispielverbindungen erfolgte mittels ¹H-NMR-Spektroskopie. Zusätzlich wurden für alle BeispielVerbindungen und ausgewählte Zwischenprodukte massenspektrometrische Untersuchungen (MS, Angabe m/z für [M+H]⁺) durchgeführt. Die NMR-Spektren wurden mit NMR-Spektrometern der Firma Bruker Analytik GmbH, Silberstreifen 4, D-76287 Rheinstetten gemessen. Die Gerätebezeichnungen lauten: für 300 MHz: Avance DPX 300 MHz, für 600 MHz: Avance DRX 600 MHz.

### Synthese der Beispielverbindungen:

Die Beispielverbindungen **1-4** sind kommerziell erhältlich (siehe vorstehende Liste kommerzieller Anbieter).

Dem Fachmann sind entsprechend Methoden zur Durchführung der Synthese von Verbindungen der allgemeinen Formel (I) geläufig, welche u.a. den Standardwerken der Organischen Chemie zu entnehmen wie bspw. J. March, Advanced Organic Chemistry, Wiley & Sons, 6th edition, 2007; F. A. Carey, R. J. Sundberg, Advanced Organic Chemistry, Parts A and B, Springer, 5th edition, 2007); Autorenkollektiv, Compendium of Organic Synthetic Methods, Wiley & Sons. Darüber hinaus können weitere Methoden sowie Literaturverweise von den gängigen Datenbanken wie bspw. der Reaxys® Datenbank der Firma Elsevier, Amsterdam, NL oder der SciFinder® Datenbank der American Chemical Society, Washington, US, ausgegeben werden.

### Beispiel 1:

2,2'-(5,5'-(1,4-Phenylenbis(oxy))bis(1,3-dioxoisoindolin-5,2-diyl))dibenzoesäure

Ein Fachmann ist in der Lage, diese und analoge Verbindungen herzustellen.

Beispiel **1** kann z.B. durch Aminolyse von 5,5'-(1,4-Phenylenbis(oxy))diisobenzofuran mit zwei Äquivalenten 2-Aminobenzoesäure in einem hoch-siedendem Lösungsmittel wie z.B. DMF hergestellt werden, wie es zu analogen Verbindungen in US 4,319,547 beschrieben ist.

### Beispiel 2:

2-(4-(5-(2-Carboxy-4-hydroxyphenylcarbamoyl)-1,3-dioxoisoindolin-2-yl)benzamido)-5-hydroxybenzoesäure

Ein Fachmann ist in der Lage, diese und analoge Verbindungen herzustellen. Beispiel **2** kann z.B. unter Schotten-Baumann-Bedingungen durch Reaktion von 2-(4-(Chlorocarbonyl)phenyl)-1,3-dioxoisoindolin-5-carbonylchlorid (gemäß I. Sava et al., Rev. Roum. Chim. 37, 1145 (1992)) mit zwei Äquivalenten 2-Amino-5-hydroxybenzoesäure in Gegenwart eines anorganischen oder organischen Säurefängers hergestellt werden.

### Beispiel 3:

2-(3-(5-(2-Carboxyphenylcarbamoyl)-1,3-dioxoisoindolin-2-yl)benzamido)-benzoesäure

Ein Fachmann ist in der Lage, diese und analoge Verbindungen herzustellen. Beispiel **3** kann z.B. unter Schotten-Baumann-Bedinungen durch Reaktion von 2-(3-(Chlorocarbonyl)phenyl)-1,3-dioxoisoindolin-5-carbonylchlorid (gemäß US 6,468,990) mit zwei Äquivalenten 2-Aminobenzoesäure in Gegenwart eines anorganischen oder organischen Säurefängers hergestellt werden.

### Beispiel 4:

2-(4-(5-(2-Carboxylato-4-hydroxyphenylcarbamoyl)-1,3-dioxoisoindolin-2-yl)-benzamido)-5-hydroxybenzoesäure, Natriumsalz

In 10 ml destilliertem Wasser werden unter Rühren 0,6 g (1 mmol) 2-(4-(5-(2-Carboxy-4-hydroxyphenylcarbamoyl)-1,3-dioxoisoindolin-2-yl)benzamido)-5-hydroxybenzosäure (Beispiel 2) aufgeschlämmt und 80 mg (2 mmol) festes Natriumhydroxid zugegeben und 750 ml Ethanol, um eine vollständige Lösung zu erreichen. Nach 30 Minuten wird das Ethanol abdestilliert und der Rückstand gefriergetrocknet. Es werden 0,676 g eines gelben Feststoffs erhalten.

¹H-NMR (d₆-DMSOext): δ [ppm] = 6,80 ppm (d, 2H, 2 x Aryl-H), J = 6,79 Hz); 7,52 ppm (s (breit), 2H, 2 x Aryl-H); 7,65 ppm (d, 2H, 2 x Aryl-H, J = 7,63 Hz); 7,85 ppm (d, 2H, 2 x Aryl-H, J = 7,85 Hz); 7,98 ppm (d, 2H, 1 x Aryl-H); 8,13 ppm (d, 1 H, 1 x Aryl-H, J = 8,12 Hz); 8,50 ppm (m, 2H, 2 x Aryl-H, J = 8,12 Hz); 10,84 ppm (s (breit), 2H, 2 x OH); 12,75 ppm (d, 2H, 2 x NH).

### Pharmakologische Daten

Die Affinität der Verbindungen der allgemeinen Formel (I) für den vGLUT1- bzw. vGLUT2-Transporter wurde wie vorstehend beschrieben bestimmt (Pharmakologische Methoden)

Die Verbindungen der vorstehend angegebenen Formel (I) weisen eine ausgezeichnet Affinität zum vGLUT1- bzw. vGLUT2-Transporter (Tabelle 1.).

In Tabelle 1 haben die nachfolgenden Abkürzungen die folgenden Bedeutungen:

Unter "IC₅₀-Fest" wird die Konzentration verstanden, bei der eine halbmaximale Inhibition beobachtet wird, wenn die Verbindung frisch aus der Festsubstanz in Lösung gebracht worden ist.

**Tabelle 1**

| **Verbindung gemäß Beispiel** | **VGLUT (Ratte), Hemmung [%]** | **VGLUT (Ratte), IC₅₀ [µM]** | **VGLUT (Ratte), ICₛₒ-Fest [µM]** |
|---|---|---|---|
| **1** | 74 | 3,81 | |
| **2** | 98 | 0,0946 | 0,032 |
| **3** | 97 | 0,56 | |
| **4** | | | 0,154 |

## Patentansprüche

1. Wenigstens eine substituierte Verbindung der allgemeinen Formel (I) 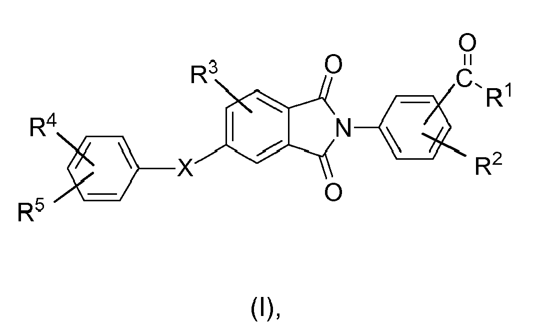 worin
X für -C(=O)NH-, -NH(C=O)- oder für 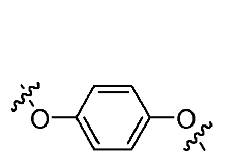 steht;
R¹ für OR⁶; oder NR⁶R⁷ steht,
wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert; C₃-₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
_{R}², _{R}³, R⁴ und R⁵ jeweils unabhängig voneinander für H; F; Cl; Br; I; OR⁸; SR⁸; C(=O)OR⁸; CN; CF₃; NO₂; NR⁸R⁹; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃-₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen,
wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert; C₃-₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,
wobei R¹⁰ für C₁₋₁₀-Alkyl, gesättigt oder ungesättigt, verzweigt oder unverzweigt, unsubstituiert oder einfach oder mehrfach substituiert; C₃-₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl verbrücktes C₃₋₁₀-Cycoalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; steht;
worin "Alkyl substituiert", "Heterocyclyl substituiert" und "Cycloalkyl substituiert" an den entsprechenden Resten für die Substitution eines oder mehrerer Wasserstoffatome jeweils durch Substituenten unabhängig voneinander ausgewählt aus der Gruppe bestehend aus F; CI; Br; I; N0₂; CN; =O; CF₃; C₁₋₈-Alkyl, Aryl; Heteroaryl; C₃₋₁₀-Cycloalkyl; Heterocyclyl; C(=O)OH;
C(=O)O-C₁₋₈-Alkyl; C(=O)O-C₃₋₁₀-Cycloalkyl, C(=O)O-Aryl; C(=O)O-Heteroaryl; C(=O)NH₂; C(=O)NH-C₁-₈-Alkyl, C(=O)NH-C₃₋₁₀-Cycoalkyl; C(=O)NH-Aryl; C(=O)NH-Heteroaryl; C(=O)N(C₁-₈-Alkyl)₂; C(=O)N(C₃₋₁₀-Cycloalkylh; C(=O)N(Aryl)₂; C(=O)N(Heteroaryl)₂; C(=O )N(C₁₋₈-Alkyl)(C₃₋₁₀-Cycloalkyl); C(=O)N(C₁-₈-Alkyl)(Aryl); C(=O)N(C₁₋₈-Alkyl)(Heteroaryl); C(=O)N(C₃₋₁₀-Cycloalkyl)(Aryl); C(=O)N(C₃₋₁₀-Cycloalkyl)(Heteroaryl); C(=O)N(Aryl)(Heteroaryl); OH; OCF₃; O-C₁-₈-Alkyl; O-C₃-₁₀-Cycloalkyl; O-Aryl; O-Heteroaryl; O-C(=O)-C₁₋₈-Alkyl, O-C(=O)-C₃₋₁₀-Cycloalkyl; O-C(=O)-Aryl; O-C(=O)-Heteroaryl; SH; SCF₃; S-C₁₋₈-Alkyl; S- C₃-₁₀-Cycloalkyl; S-Aryl; S-Heteroaryl; NH₂; NH-C₁₋₈-Alkyl; NH-C3₋₁₀-Cycloalkyl; NH-Aryl; NH-Heteroaryl; N(C₁₋₈-Alkyl)₂; N(C₃₋₁₀-Cycoalkyl)₂; N(Aryl)₂; N(Heteroaryl)₂; N(C₁₋₈-Alkyl)(C₃-₁₀-Cycloalkyl); N(C₁₋₈-Alkyl)(Aryl); N(C₁₋₈-Alkyl)(Heteroaryl); N(C₃₋₁₀-Cycloalkyl)(Aryl); N(C₃-₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl; steht;
worin "Aryl substituiert" und "Heteroaryl substituiert" an den entsprechenden Resten für die Substitution eines oder mehrerer Wasserstoffatome jeweils durch Substituenten unabhängig voneinander ausgewählt aus der Gruppe bestehend aus F; CI; Br; I; NO₂; CN; CF₃; C₁₋₈-Alkyl; Aryl; Heteroaryl; C₃₋₁₀-Cycloalkyl; Heterocyclyl; C(=O)OH; C(=O)O-C₁₋₈-Alkyl; C(=O)O-C₃₋₁₀-Cycloalkyl; C(=O)O-Aryl; C(=O)O-Heteroaryl; C(=O)NH₂; C(=O)NH-C₁₋₈-Alkyl; C(=O)NH-C₃₋₁₀-Cycloalkyl; C(=O)NH-Aryl; C(=O)NH-Heteroaryl; C(=O)N(C₁₋₈-Alkyl)₂; C(=O)N(C₃₋₁₀-Cycloalkyl)₂; C(=O)N(Aryl)₂; C(=O)N(Heteroaryl)_{2;} C(=O)N(C₁₋₈-Alkyl)(C₃-₁₀-Cycloalkyl); C(=O)N(C₁₋₈-Alkyl)(Aryl); C(=O)N(C₁₋₈-Alkyl)(Heteroaryl); C(=O)N(C₃₋₁₀-Cycloalkyl)(Aryl); C(=0)N(C₃-₁₀-Cycloalkyl)(Heteroaryl); C(=O)N(Aryl)(Heteroaryl); OH; OCF₃; O-C₁₋₈-Alkyl; O-C₃-₁₀-Cycloalkyl; O-Aryl; O-Heteroaryl; O-C(=O)-C₁₋₈-Alkyl; O-C(=O)-C₃₋₁₀-Cycloalkyl; O-C(=O)-Aryl; 0-C(=O)-Heteroaryl; SH; SCF₃; S-C₁₋₈-Alkyl; S- C₃₋₁₀-Cycloalkyl; S-Aryl; S-Heteroaryl; NH₂; NH-C₁-₈-Alkyl; NH-C₃₋₁₀-Cycloalkyl; NH-Aryl; NH-Heteroaryl; N(C₁₋₈-Alkyl)₂; N(C₃₋₁₀-Cycoalkyl)₂; N(Aryl)₂; N(Heteroaryl)₂; N(C₁₋₈-Alkyl)(C₃₋₁₀-Cycloalkyl); N(C₁₋₈-Alkyl)(Aryl); N(C₁₋₈-Alkyl)(Heteroaryl); N(C₃₋₁₀-Cycloalkyl)(Aryl); N(C₃₋₁₀-Cycloalkyl)(Heteroaryl); N(Aryl)(Heteroaryl); NH-C(=O)-C₁₋₈-Alkyl; NH-C(=O)-C₃₋₁₀-Cycloalkyl; NH-C(=O)-Aryl und NH-C(=O)-Heteroaryl steht;
jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;
zur Verwendung als Arzneimittel.

2. Wenigstens eine substituierte Verbindung (I) gemäß Anspruch 1, wobei
X für -NH(C=O)- oder für 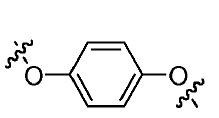 steht, zur Verwendung als Arzneimittel.

3. Wenigstens eine substituierte Verbindung (I) gemäß Anspruch 1 oder 2, wobei R¹ für OR⁶; oder NR ⁶R⁷ steht,
wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder
Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; stehen,
zur Verwendung als Arzneimittel.

4. Wenigstens eine substituierte Verbindung (I) gemäß einem der Ansprüche 1 - 3, wobei
R¹ für OR⁶ oder NR⁶R⁷steht,
wobei R⁶ und R⁷ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl und NH₂; Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; über C₁₋₈-Alkyl verbrücktes Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂, wobei die Alkylkette unsubstituiert ist und jeweils verzweigt oder unverzweigt, gesättigt oder ungesättigt; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂ und F, wobei die Alkylkette unsubstituiert ist und jeweils verzweigt oder unverzweigt, gesättigt oder ungesättigt sein kann; stehen,
zur Verwendung als Arzneimittel.

5. Wenigstens eine substituierte Verbindung (I) gemäß einem der Ansprüche 1 - 4, wobei
R² und R³ jeweils unabhängig voneinander für H; F; CI; Br; I; OR⁸; C(=O)OR⁸; CF₃; NR⁸R⁹; C₁₋₁₀-Alkyl, gesättigt oder ungesättigt,
verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,
wobei R⁸ und R⁹ jeweils unabhängig voneinander für H; C₁-₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃-₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder
Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I;, wobei die Alkylkette unsubstituiert ist und jeweils verzweigt oder unverzweigt, gesättigt oder ungesättigt sein kann; stehen,
zur Verwendung als Arzneimittel.

6. Wenigstens eine substituierte Verbindung (I) gemäß einem der Ansprüche 1 - 5, wobei
R² und R³ jeweils unabhängig voneinander für H; F; Cl, Br; I; OH; C(=O)OH; CF₃; C₁₋₁₀-Alkyl, gesättigt oder ungesättigt, verzweigt oder unverzweigt, unsubstituiert; Phenyl, unsubstituiert; oder über C₁₋₈-Alkyl-verbrücktes Phenyl, jeweils unsubstituiert, stehen,
zur Verwendung als Arzneimittel.

7. Wenigstens eine substituierte Verbindung (I) gemäß einem der Ansprüche 1 - 6, wobei
R⁴ und R⁵ jeweils unabhängig voneinander für H; F; Cl, Br; I; OR⁸; C(=O)OR⁸; CF_{3;} NR⁸R⁹;C₁₋₁₀ -Alkyl, gesättigt oder ungesättigt,
verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃-₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,
wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃-₁₀-Cycloalkyl oder
Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette unsubstituiert ist und jeweils verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen;
oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,
wobei R¹⁰ für C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl, NH₂, F, Cl, Br und I; C₃-₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I, wobei die Alkylkette jeweils unsubstituiert ist und verzweigt oder unverzweigt, gesättigt oder ungesättigt sein kann; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, CI, Br und I, wobei die Alkylkette unsubstituiert ist und jeweils verzweigt oder unverzweigt, gesättigt oder ungesättigt sein kann; steht,
zur Verwendung als Arzneimittel.

8. Wenigstens eine substituierte Verbindung (I) gemäß einem der Ansprüche 1 - 7, wobei
R⁴ und R⁵ jeweils unabhängig voneinander für H; F; CI; Br; I; OH; C(=O)OH; CF₃, C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,
oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,
wobei R¹⁰ für C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; steht,
zur Verwendung als Arzneimittel.

9. Wenigstens eine substituierte Verbindung (I) gemäß einem der Ansprüche 1 - 8, wobei
X für -NH(C=O)- oder für 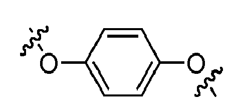 steht, R¹ für OR⁶ oder NR⁶R⁷ steht,
wobei R⁷ für H steht und R⁶ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, OH, O-C₁₋₄-Alkyl und NH₂; Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂; über C₁₋₈-Alkyl verbrücktes Cyclohexyl, Pyrrolidinyl, Piperidinyl, Piperazinyl oder Morpholinyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl und NH₂, wobei die Alkylkette unsubstituiert ist und jeweils verzweigt oder unverzweigt, gesättigt oder ungesättigt sein kann; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂ und F, wobei die Alkylkette unsubstituiert ist und jeweils verzweigt oder unverzweigt, gesättigt oder ungesättigt sein kann; stehen;
R² und R³ jeweils unabhängig voneinander für H; F; CI; Br; I; OH; C(=O)OH; CF₃; C₁₋₁₀-Alkyl, gesättigt oder ungesättigt, verzweigt oder unverzweigt, unsubstituiert; Phenyl, unsubstituiert; oder über C₁₋₈-Alkyl-verbrücktes Phenyl, jeweils unsubstituiert, stehen,
R⁴ und R⁵ jeweils unabhängig voneinander für H; F; CI; Br; I; OH;
C(=O)OH; CF₃; C₁-₁₀-Alkyl, gesättigt oder ungesättigt, verzweigt oder unverzweigt, unsubstituiert oder einfach oder mehrfach
substituiert mit einem oder mehreren Substituenten jeweils unabhängig voneinander ausgewählt aus der Gruppe bestehend aus, C(=O)OH, OH, O-C₁-₄-Alkyl, NH₂, F, Cl, Br und I; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; stehen,
oder, wenn R⁴ und R⁵ an einander benachbarte Kohlenstoffatome gebunden sind, R⁴ und R⁵ gemeinsam für die Gruppe -C(=O)-NR¹⁰-C(=O)- stehen können,
wobei R¹⁰ für C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus, C(=O)OH, OH, O-C₁₋₄-Alkyl, NH₂, F, Cl, Br und I; Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; oder über C₁₋₈-Alkyl-verbrücktes Phenyl, Naphthyl oder Pyridyl, jeweils unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, OH, O-C₁₋₄-Alkyl, NH₂, F, Cl, Br und I; wobei die Alkylkette unsubstituiert ist und jeweils verzweigt oder unverzweigt, gesättigt oder ungesättigt sein kann; steht,
zur Verwendung als Arzneimittel.

10. Wenigstens eine substituierte Verbindung (I) gemäß einem der Ansprüche 1-9 ausgewählt aus der Gruppe
1 2,2'-(5,5'-(1,4-Phenylenbis(oxy))bis(1,3-dioxoisoindolin-5,2-diyl))dibenzoesäure;
2 2-(4-(5-(2-Carboxy-4-hydroxyphenylcarbamoyl)-1,3-dioxoisoindolin-2-yl)benzamido)-5-hydroxybenzoesäure;
3 2-(3-(5-(2-Carboxyphenylcarbamoyl)-1,3-dioxoisoindolin-2-yl)benzamido)-benzoesäure und
4 2-(4-(5-(2-Carboxylato-4-hydroxyphenylcarbamoyl)-1,3-dioxoisoindolin-2-yl)-benzamido)-5-hydroxybenzoesäure,
jeweils in Form der freien Verbindungen und/oder in Form der Salze physiologisch verträglicher Säuren oder Basen,
zur Verwendung als Arzneimittel.

11. Wenigstens eine substituierte Verbindung (I) wie in einem der Ansprüche 1-10 beschrieben,
jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;
zur Behandlung und/oder Prophylaxe von einer oder mehreren Erkrankungen ausgewählt aus der Gruppe umfassend:
Schmerz, akuter Schmerz, chronischer Schmerz, neuropathischer Schmerz,
visceraler Schmerz, inflammatorischer Schmerz, Gelenkschmerz, Hyperalgesie, Allodynie, Kausalgie, Migräne,
neurodegenerative Erkrankungen, Multiple Sklerose, Morbus Alzheimer,
Morbus Parkinson, Morbus Huntington, amyotrophe Lateralsklerose,
kognitive Dysfunktionen, kognitive Mangelzustände, Epilepsie,
Störungen der Nahrungsaufnahme, Bulimie, Kachexie, Anorexie,
Fettleibigkeit;
Störung des visuellen Systems, Schizophrenie, Diabetes und/oder Depressionen; und/oder
zur Lokalanästhesie; und/oder
zur Hemmung unerwünschter Nebenwirkungen, Übelkeit, Erbrechen, Abhängigkeit, Atemdepression und/oder Obstipation.

12. Verwendung wenigstens einer substituierten Verbindung der allgemeinen Formel (I) gemäß Anspruch 1, in der X, R¹, R², R³, R⁴, R⁵, R⁶, R⁷, R⁸, R⁹ und R¹⁰ die Bedeutung gemäß einem der Ansprüche 1 bis 10 haben,
jeweils vorliegend in einer Form ausgewählt aus der Gruppe umfassend die freie Verbindung, das Racemat, ein Enantiomer, ein Diastereomer, eine Mischung von Enantiomeren, Diastereomeren oder eines einzelnen Enantiomers oder Diastereomers und/oder in Form eines Salzes physiologisch verträglicher Säuren oder Basen;
zur Herstellung eines Arzneimittels zur Behandlung und/oder Prophylaxe von einer oder mehrerer Erkrankungen ausgewählt aus der Gruppe umfassend:
Schmerz, akuter Schmerz, chronischer Schmerz, neuropathischer Schmerz,
visceraler Schmerz, inflammatorischer Schmerz, Gelenkschmerz, Hyperalgesie, Allodynie, Kausalgie, Migräne,
neurodegenerative Erkrankungen, Multiple Sklerose, Morbus Alzheimer,
Morbus Parkinson, Morbus Huntington, amyotrophe Lateralsklerose,
kognitive Dysfunktionen, kognitive Mangelzuständen, Epilepsie,
Störungen der Nahrungsaufnahme, Bulimie, Kachexie, Anorexie,
Fettleibigkeit;
Störung des visuellen Systems, Schizophrenie, Diabetes und/oder Depressionen; und/oder
zur Lokalanästhesie; und/oder
zur Hemmung unerwünschter Nebenwirkungen, Übelkeit, Erbrechen, Abhängigkeit, Atemdepression und/oder Obstipation.

13. Arzneimittel, enthaltend wenigstens eine substituierte Verbindung der allgemeinen Formel (I) gemäß Anspruch 1, in der X, R¹, R², R³, R⁴, R⁵, R⁶, _{R}⁷, R⁸, R⁹ und R¹⁰ die Bedeutung gemäß einem der Ansprüche 1 bis 10 haben.
